Europäisches Patentamt

European Patent Office

Office européen des brevets

(11) Publication number: **0 320 023 B1**

# EUROPEAN PATENT SPECIFICATION

(45) Date of publication of patent specification: **21.09.94**

(51) Int. Cl.5: **B01D 15/00**, B01D 15/08, G01N 30/48

(21) Application number: **88120747.6**

(22) Date of filing: **12.12.88**

(54) **Macroporous polymeric membranes, their preparation and their use for polymer separation.**

(30) Priority: **10.12.87 CS 9034/87**
**21.10.88 CS 6987/88**

(43) Date of publication of application:
**14.06.89 Bulletin 89/24**

(45) Publication of the grant of the patent:
**21.09.94 Bulletin 94/38**

(84) Designated Contracting States:
**BE DE FR GB NL SE**

(56) References cited:
**EP-A- 0 186 758**
**WO-A-82/01553**
**US-A- 3 926 864**
**US-A- 3 928 255**
**US-A- 4 267 295**

**PATENT ABSTRACTS OF JAPAN vol. 13, no. 158 (C-586)(3506), 17 April 1989; & JP - A - 63315145 (TERUNO CORP.) 22.12.1988**

(73) Proprietor: **Ceskoslovenska akademie ved**
**Narodni trida 3**
**Praha 1 (CS)**

Proprietor: **AKADEMIA NAUK SSSR**
**Leninskij Prospekt 14**
**Moskva (RU)**

(72) Inventor: **Svec, Frantisek, Dipl.-Ing., Dr.sc.**
**Pricna 26**
**CS-Hrebec (CS)**
Inventor: **Bleha, Miroslav, Dipl.-Ing.CSc**
**Lublanska 46**
**CS-Praha-2 (CS)**
Inventor: **Tennikova, Tatiana Borisovna CSc**
**Mitninskaja 18**
**SU-Leningrad (SU)**
Inventor: **Belenkii, Boris Grigorijevic DrSc**
**Litejnyj prospekt 46**
**SU-Leningrad (SU)**

(74) Representative: **Patentanwälte Beetz - Timpe - Siegfried Schmitt-Fumian - Mayr**
**Steinsdorfstrasse 10**
**D-80538 München (DE)**

**Description**

The invention relates to macroporous polymeric membranes, a method for their preparation and to their application to the separation of polymers.

Although the separation of macromolecules from their mixtures with both low-molecular weight and high-molecular weight compounds are very often discussed in the present literature and practice, and considerable improvements could be attained, many problems could not be solved. The problem of an efficient separation of a product from a reaction medium becomes a decisive criterion for a successful method in many modern, fast developing scientific and technical disciplines, e.g., in biotechnology. As a rule, it is not very difficult to find a method for the analytical identification of a desired compound or to determine its concentration. However, difficulties arise from the still incompletely solved problem of preparative industrial separation with high efficiency and a reasonable expenditure of labour, energy, capital and material resources and at minimum environmental load.

The separation and isolation of biopolymers is most important from the aspects of their application. This group of macromolecular compounds comprises particularly oligopeptides and polypeptides, proteins, enzymes, lectins, antibodies, antigens, nucleic acids, and polysaccharides. The separation of biopolymers from natural sources has been the generally known problem already since last century.

The first purification methods were based above all on precipitation, e.g., of proteins, or on salting out with neutral salts, which may be carried out also with simultaneous variation of pH for obtaining even a multiple fractionation in one step. But nevertheless, it was a long way until a pure protein could be obtained, and one of the first successes was in 1926, when Sumner isolated crystalline urease.

The method of chromatography (Ber. Deut. Botan. Ges. 24 (1906) 316) developed in parallel. However, first the work of A.J.P. Martin and R.L.M. Synge (Biochem. J., 35, 1358, 1941), who introduced the concept of the theoretical plate into the liquid chromatography, became an important landmark. It was also stated there that columns packed with microparticles are especially suitable for the separation of macromolecules, the diffusion coefficients of which are very low. Since microparticles were not yet available at that time, the high-performance liquid chromatography (HPLC) was realized first twenty years later.

The chromatography of polymers developed first on the basis of the findings of Peterson and Sober (J. Amer. Chem. Soc. 76 (1954) 1711) that proteins may be adsorbed on the diethylaminoethyl derivative of cellulose and then gradually eluted by a solution with increasing ionic strength. Later-on, also other cellulose derivatives were used for the same purpose, e.g., carboxymethyl cellulose. At the end of the fifties, cross-linked dextran gels were introduced for size-exclusion chromatography (SEC) of proteins and nucleic acids (DE-B-1 292 883; GB-B-974 054), where the separation occurs on the basis of the accessability of various portions of the gel structure for macromolecular species of different size. However, the low mechanical strength of gel media did not enable the full realization of the HPLC principle.

A breakthrough occured in the fractionation of bacitracin on microparticles of silica derivatized with alkyl silanes (K. Tsuji, J.H. Robinson, J. Chromatog. 112 (1976) 663). Several structure forms of this peptide were separated by the method of so-called reversed-phase liquid chromatography (RPLC) in a column of a length of 30 cm using an acidic mobile phase containing an organic solvent. Soon after that, papers also appeared describing the application of silica for high-performance size-exclusion chromatography (SEC) and ion-exchange liquid chromatography (IELC) of proteins with almost quantitative yield and retained activity. In comparison with gel materials, flow rates of the mobile phase almost two orders of magnitude higher can be used in these cases and thus substantially reduce the time of analysis.

Generally, macromolecules interact with the stationary phase in a chromatographic column in various ways. This has the consequence that the capacity factor k' defined for an isocratic elution, i.e. elution using the same solvent all the time, given by

$$k' = (t_R - t_o)/t_o,$$

wherein $t_R$ is the retention time of the desired compound, and $t_o$ is the column dead time, drastically changes at a slight change in the eluent composition. At certain compositions, k' is so high that the macromolecule practically does not move in the column. However, a small change in the solvent properties causes a decrease in k' to a value close to zero, and the macromolecule passes through the column without any interaction with the packing. The usually measured dependence of log k' on the eluant composition is thus very steep, and often almost vertical. Consequently, the length of the chromatographic column has not a decisive influence on the quality of separation. Very short columns may be used containing only such an amount of sorbent which is necessary for the sorption of the separated molecules in the amount required for the detection of the components after separation.

2

It revealed that in the chromatography of macromolecules, e.g., by the RPLC method, the resolution function is proportional to

$$D_m^{1/2} \cdot d_p^{-1} \cdot t_G^{1/2} \quad ,$$

where $D_m$ is the diffusion coefficient of the solute, $d_p$ is the diameter of the particles packed into the column, and $t_G$ is the time during which the composition of the solvent mixture varies (gradient). Because the first quantity is a characteristic constant of the separated compound, the quality of separation may be improved by a small change in the solvent composition or by decreasing the size of the particles of the column packing. In the first case, the time necessary for the separation of the mixture increases, whereas in the second case the pressure loss in the column increases, and the eluent has to be introduced under a very high pressure, which may reach several or several tens of MPa.

The given facts show that an enlargement of the scale of a chromatographic separation from an analytical or laboratory scale to an industrial scale brings up immense problems. It is obvious that the application of large-volume columns of a relatively large diameter may cause difficulties, even if incompressible packings are used; this may be seen from the fact that the separation results are not comparable to those obtained on a small scale. The so-called tailing of chromatographic peaks or peak overlapping occur very often. The reason may be the different flow rate of eluent at various sites of the cross-section of column. In order to avoid this effect, a uniform propagation rate of the (horizontal) front has to be attained throughout the column in the direction from the inlet of eluent to the column outlet. This problem is discussed in US-A-3 250 058, and for overcoming it breakers fitted inside the column are proposed.

Methods for influencing the flow of liquid in a column are also subject-matter of other patents (US-A-3 539 505; JP-B-73-68 752) which enable to increase the scale of separations, but to the detriment of the original simplicity. In order to overcome the problems involved with complex column design, some authours focussed to the packing as such (US-A-3 856 681) or special methods for column packing (US-A-4 211 656). It revealed later that a suitable effect can be obtained if small particles of the sorbent used for separation are incorporated into a porous inert matrix having a fibrous form. This fibrous material is then packed into a column of special design, which than exhibits a far lower pressure loss and zone spreading (US-A-4 384 957, 4 512 897, 4 496 461 and 4 604 198).

As indicated above, most of the packings of chromatographic columns for the separation of biopolymers are porous materials on the basis of inorganic polymers (silica gel, glass) or organic polymers (styrene - divinylbenzene, acrylate or methacrylate copolymers, etc.), usually in the form of spherical particles (F.E. Renier, Chromatographia 24 (1987) 241). These particles are prepared predominantly by a suspension technique, and, recently, also by the multiste dispersion method (seeded polymerization). On completion of the polymerization, except a few cases, a narrow-size fraction of the sorbent has to be obtained from the raw product, since the quality of the packed column and, consequently, also its efficiency strongly depend on the particle size distribution. The fractionation of particles is very tedious, and the fraction of particles applicable for HPLC is only a small part of the entire quantity of raw product.

In view of the application of small-diameter particles (5 - 10 $\mu$m), also the organic sorbents have to be sufficiently mechanically resistant, which requires a relatively high concentration of a cross-linking agent in the polymerization batch. The requirement of porosity is met at the same time by the synthesis of macroporous particles, i.e. particles which exhibit porosity also in the dry state or in thermodynamically poor solvents (CS-B-168 268, GB-B-1 512 462), CA-A-1 049 194). From the aspects of morphology, the macroporous polymer particles are characterized by a globular microstructure, i.e. the particles consist of mutually connected submicroscopic spherical entities called globules. The specific surface area of these macroporous polymers is then, as a matter of fact, the surface area of these globules, and the interstitial spaces between them are pores (see, e.g., Z. Pelzbauer et al., J. Chromatog. 171 (1979) 101). The globular structure of porous particles suggests to some extent a spherical body filled with small, also spherical globules. Such a suggestion is not far from the conditions occuring inside a packed chromatographic column, which, however, has the shape of a cylinder. Provided the column is packed with particles having the shape of globules of 0,1 to 0,4 $\mu$m in size, there will be no practical utility for the chromatographic separation, because the pressure to be applied would be unrealistically high.

Membranes for electrodialysis, which have only the surface layer consisting of a macroporous polymer, are described e.g. in US-A-3 926 864. Such a surface has, after modification with ionogenic groups, significant anti-fouling properties. Because also suitable electrodialytic properties need to be achieved, the polymerization is carried out in such a way that the inner part of the membranes is microporous (formed by a gel). For this reason, these membranes cannot be used for the separation of polymers. They are suitable for the desalination of water, the removal of ions, and the like.

There are also known plates for thin-layer chromatography, where, however, the sorbent is deposited in the form of a layer on a solid, non-porous substrate (glass, metal). The layer is used in the separation process, which comprises chromatographic separation in tangential direction, i.e., in a path the length of which considerably exceeds the particle size of the sorbent. A further disadvantage of thin layers, which are loosely poured or bonded, is the low resistance to mechanical damage. Accordingly, thin-layer chromatography can be utilized for preparative purposes only with large difficulties.

The above given survey on the state of the art clearly shows that reliable and simple methods for the separation of polymers in larger scale are not available. Therefore, it is the object of the present invention to find a general new solution and to provide macroporous polymeric membranes suitable above all for the separation of polymers, a method for preparing these membranes and their use, including an apparatus for the chromatographic separation on the basis of the macroporous polymeric membranes.

The above object is achieved according to the independent claims. The dependent claims relate to preferred embodiments.

The macroporous polymeric membranes according to the present invention, which are particularly provided for the separation of macromolecular substances,

- consist of a cross-linked polymer or copolymer on the basis of one or more monovinylic monomers selected from

  acrylates, particularly glycidyl acrylate,
  methacrylates, particularly glycidyl methacrylate,
  itaconates, particularly glycidyl itaconate,
  vinylpyridine,
  N-vinyl pyrrolidone,
  vinyl acetate,
  glycidyl vinyl ether,
  glycidyl vinyl adipate, and
  hydroxystyrene,

  and one or more cross-linking agents selected from alkylene diacrylates, particularly ethylene diacrylate, alkylene dimethacrylates, particularly ethylene dimethacrylate, hydroxyalkylene diacrylates, hydroxyalkylene dimethacrylates, divinyl pyridine and divinyl benzene,

- and have a globular

  microstructure wherein the globular structures have a size of 0,05 to 0,5 $\mu$m and are mutually interconnected by covalent bonds, and between the globular structures being mutually communicating voids.

The macroporous membranes according to the invention preferably have a total cross-section thickness of 0,2 to 15 mm, and the specific surface area, which is measurable also in the dry state, is up to 400 m$^2$/g.

The mass ratio of monovinylic monomers(s) and the cross-linking agent(s) is preferably within the range of from 5:95 to 95:5.

The membranes may contain a reinforcing material or insert in order to increase their mechanical strength, preferably throughout the whole cross-section.

Glycidyl methacrylate is advantageously used as the monovinylic monomer in an amount of 5 to 80 % by volume, based on the total volume of monomers including cross-linking agent(s) in the batch, while the residual part of 20 to 95 % by volume is formed by the cross-linking agent and particularly a divinyl monomer, advantageously ethylene dimethacrylate. It is obvious that arbitrary mixtures of both types of monomers may be used, and, in this way, the porosity properties of the formed membranes may be varied.

Suitable cross-linking agents may be chosen from diacrylates and dimethacrylates having their vinyl groups connected by ester bonds with a chain of various length or of various hydrophilicity.

The membranes according to the invention may advantageously be produced by

- placing a monomer mixture of one or more monovinylic monomers selected from

  acrylates, particularly glycidyl acrylate,
  methacrylates, particularly glycidyl methacrylate,
  itaconates, particularly glycidyl itaconate,
  vinylpyridine,
  N-vinyl pyrrolidone,
  vinyl acetate,

4

glycidyl vinyl ether,
glycidyl vinyl adipate, and
hydroxystyrene,
and one or more cross-linking agents selected from
alkylene diacrylates, particularly ethylene diacrylate,
alkylene dimethacrylates, particularly ethylene dimethacrylate,
hydroxyalkylene diacrylates,
hydroxyalkylene dimethacrylates,
divinyl pyridine and
divinyl benzene,
and a polymerization initiator, preferably a radical polymerization initiator,
dissolved in a porogenic inert organic solvent in a mould comprising two temperature-controlled, parallel face plates facing each other and a distance insert having a thickness corresponding to the required thickness of the membrane and being adapted to be in a sealing contact with the face plates, size and shape of the inner space of the mould defined by the face plates and the distance insert being adapted to size and shape of the desired membrane, and

- polymerizing or copolymerizing, respectively, with heating the face plates to the polymerization or copolymerization temperature for the necessary reaction duration.

The polymerization of the mixture in the mould is usually carried out by heating to a temperature of up to 80 °C for ≤ 24 h, whereby globules are formed during polymerization with a suitable combination of monomer, cross-linking agent, and inert porogenic solvent, which increase their size with increasing conversion as far as they come into contact and mutual bonding occurs, thus providing the membrane with the desirable mechanical strength.

Azo-bis(isobutyronitrile) is preferably used in an amount of 0,05 to 2 % by mass, based on the total amount of monomers, in order to initiate the radial polymerization; however, also other radical initiators may be used which may be selected e.g. from azo compounds, peroxides, hydroperoxides, redox systems, and the like.

An important component of the polymerizing system is the porogenic solvent, which is preferably used in the polymerization batch in an amount of 40 to 60 % by volume. Cyclohexanol or its mixtures comprising up to 20 % by volume of dodecanol can be advantageously used. Of course, also other porogenic agents may be used which may be selected from aliphatic and aromatic alcohols, esters, ethers, ketones, hydrocarbons, silicone oils, low-molecular weight polymers, and others.

The polymeric porous membranes prepared according to the invention from reactive monomers can be further modified, and accordingly, their properties can be varied within a broad range. In this way, e.g., the hydrophilicity or lyophilicity may be increased, ionogenic groups may be introduced, and catalysts, affinants, or other active groups or molecules can be bound and immobilized.

For example, allyl, amine, sulfonate, hydrogen sulfonate, hydroxy, thiol, and alkyl groups with a chain length of up to 18 carbon atoms may be covalently bound to the inner surface of the membrane by chemical modification.

A fundamental object of this invention is also the application of the above described macroporous membranes, particularly for the separation and fractionation of macromolecular substances and particularly of synthetical polymers and biopolymers.

The general principle of the separation of macromolecular substances on the basis of the macroporous membranes of the invention may be characterized by passing a solution of the macromolecular substances to be separated under pressure through a macroporous polymeric membrane according to one of claims 1 to 7, with sorption of the macromolecular substances in the membrane, eluting with an elution solvent the properties of which are changed gradually or stepwise in such a manner that individual components of the sorbed macromolecular substances are eluted, and detecting and/or collecting individual eluted components.

In the elution step the pH, the ionic strength, the temperature and/or the composition of the elution solvent are changed, preferably according to a predetermined program.

In accordance with a preferred embodiment, a macroporous polymeric membrane according to the invention is placed and secured in a fixed position on a support member forming a part of the walls of a vessel comprising a liquid chamber provided with means for applying a pressure and optionally also means for stirring above the membrane, and a liquid collection system below the membrane connected to a liquid detection and/or collection system, the solution of the macromolecular substances is then charged into the liquid chamber, pressure is applied to the liquid chamber, and the macromolecular components or fractions are eluted and/or collected.

Usually the pressure is ≤ 1 MPa, while the polymer(s) are sorbed within the membrane. The solvent in which the polymers are dissolved is introduced into the chamber by a pump, and then the properties of the solvent are changed according to the specific program in such a way, that individual components of the sorbed polymer are gradually redissolved and eluted from the membrane and thus are separated. The course of the separation is either only detected with a suitable equipment, and the composition of the polymer or polymer mixture is qualitatively or quantitatively evaluated, or individual fractions are collected yielding the individual components of the separated mixture (preparative separation).

The programmed change of properties of the solvent which causes the gradual dissolution of individual components of the mixture (gradient) may concern the pH, the ionic strength, the content of organic solvent, temperature, and other variables, as mentioned above.

The macroporous polymeric membranes and the method of separation of polymers and particularly of biopolymers according to the present invention have numerous advantages in comparison with the present state of the art. Above all, the membrane preparation is very simple and is not limited with respect to their dimensions. The membranes have sufficient mechanical stability and resistance to physical and chemical effects. The mechanical strength may be further increased, if desired, incorporation of a reinforcing material or insert into the membrane during polymerization. Because the membranes contain reactive groups, they may be readily chemically modified, and functional groups changing their properties may be introduced onto their inner surface, thus substantially enlarging their applicability.

For example, the separation of biopolymers proceeds, in comparison to earlier methods, very fast at a considerably higher load of the separating element than is possible for chromatographic columns. At the same time, the pressure to be applied in order to obtain the required flow rate is by one to two orders of magnitude lower than in column methods of comparable efficiency. A principal advantage of the membranes and the method of their application according to the invention is, however, the possibility to use theoretically unlimitedly large areas on which the separation of polymers occurs and thus to realize even an industrial process for separating individual macromolecular compounds from mixtures. The required membrane may not only be obtained by one membrane and one chamber, but it is possible to combine several smaller membranes and chambers to blocks having the same desired total membrane area.

The invention is further explained by way of examples.

### Example 1

A mould formed from two metallic plates with drilled communication channels and a distance insert of a square shape and of a thickness of 1,2 mm made from silicon rubber, in which a square window with a side length of 8 cm and a hole enabling to charge the mould space from outside were cut out, was charged with a polymerization mixture consisting of 0,6 g of N-vinyl pyrrolidone, 11,4 g of ethylene diacrylate, 8,2 g of 2-butanone, and 0,15 g of azo-bis(isobutyronitrile) (polymerization initiator). Water of 80 °C was led into the mould channels for 8 hours. On completion of the polymerization, the mould was disassembled, and the prepared membrane having a specific surface area of 8,4 m$^2$/g was ready to use. The size of the individual globular structures, as determined by electron microscopy, was 0,48 $\mu$m.

### Example 2

The same mould as in example 1 was charged with a mixture consisting of 0,6 g of glycidyl methacrylate, 11,4 g of ethylene dimethacrylate, 0,12 g of initiator, and 16,2 g of cyclohexanol, and the polymerization was carried out under the same conditions. The prepared membrane had a specific surface area of 139 m$^2$/g; the globular structures had a diameter of 0,05 $\mu$m.

### Example 3

A mould, the distance insert of which was 15 mm in thickness, was charged with a mixture consisting of 12 ml of 2-hydroxyethyl acrylate, 12 ml of 2-hydroxypropylene diacrylate, 0,24 g of azo-bis(isobutyronitrile), 32,4 ml of cyclohexanol, and 3,6 ml of dodecanol. The polymerization was carried out for 3 hours at 60 °C and then for 5 hours at 80 °C. The obtained polymeric sheet had a specific surface area of 38 m$^2$/g; the monodisperse globules had a diameter of 0,12 $\mu$m.

**Example 4**

A mould, the distance insert of which had a thickness of 3 mm and was provided with a cut circular window with a radius of 4 cm, was charged with a mixture consisting of 4,8 g of 4-hydroxystyrene, 6,2 g of 2,3-dihydroxybutylene dimethacrylate, 0,15 g of azo-bis(isobutyronitrile), and 12,8 g of methyl benzoate. The polymerization was carried out at 80 °C for 24 hours. The final product in the form of a circular sheet with a diameter of 8 cm had a specific surface area of 12,2 $m^2$/g. The globules had a size of 0,32 $\mu$m.

**Example 5**

The same mould as in example 4 was charged with a solution of 7,2 ml of glycidyl methacrylate, 4,8 ml of ethylene dimethacrylate, and, 0,12 g of azo-bis(isobutyronitrile) in a mixture of 16,2 ml of cyclohexanol and 1,8 ml of dodecanol. After 8 hours of polymerization carried out at 70 °C, a membrane was obtained with a specific surface area of 43,3 $m^2$/g, which consisted of globules with a size of 0,16 $\mu$m.

**Example 6**

A gauze made from polyester fiber with a mesh size of about 300 $\mu$m was inserted into a mould comprising a distance insert of 3 mm thickness. The mould, the cavity of which had a square section with a side length of 60 cm, was charged with a mixture containing 19 % of 2-vinylpyridine, 19 % of 2,4-divinylpyridine, 2 % of azo-bis(isobutyronitrile), and 60 % of cyclohexanol. The polymerization was completed at 80 °C after 18 hours, and the resulting product had a specific surface area of 18,9 $m^2$/g.

**Example 7**

A membrane obtained according to example 2 was immersed into a solution of potassium hydroxide in water with a concentration of 1 mol/l at 60 °C for 18 hours. After washing the membrane with a 0,5 ml/l solution of hydrochloric acid and then with water, it contained carboxylic groups in an amount of 1,4 mmol/g.

**Example 8**

A membrane obtained according to example 5 was heated for 3 hours in a 0,5 mol/l solution of sulfuric acid. The hydrolytic reaction led to a complete cleavage of the epoxy groups to vicinal hydroxy groups and thus to an increased hydrophilicity of the membrane.

**Example 9**

A membrane obtained according to example 5 was stirred with a 50 % aqueous solution of trimethylammonium chloride for 10 hours at 80 °C. After washing with a 0,5 mol/l aqueous solution of sodium hydroxide and then with water, the content of quaternary ammonium groups determined in the product by titration was 1,93 mmol/l. The product contained 2,70 % of nitrogen according to elemental analysis.

**Example 10**

A membrane obtained according to example 8 was stirred with a 20 % solution of hexadecanoic acid chloride in benzene. After 6 hours of heating to 60 %, washing with benzene, methanol, and ether, and subsequent drying, the mass of the product was increased by 4,2 %, as compared with the initial mass, which corresponded to a 32 % conversion of epoxy groups originally present.

**Example 11**

A mould formed from two metallic plates with drilled communication channels and a 1 mm thick distance insert of square shape made from silicon rubber, in which a square window with a side length of 8 cm and a hole enabling to fill the mould from outside were cut out, was charged with a polymerization mixture consisting of 6 ml of glycidyl methacrylate, 6 ml of ethylene dimethacrylate, 0,12 g of azo-bis-(isobutyronitrile), 16,2 ml of cyclohexanol, and 1,8 ml of dodecanol. Water of 70 °C was introduced into the mould channels for 8 hours. After the polymerization was completed the mould was allowed to cool down and disassembled. The obtained sheet of the macroporous membrane was thoroughly washed with alcohol, water and again with alcohol, and allowed to dry. A circular sheet with a diameter of 10 mm was cut out therefrom with a stamp (surface area approx. 300 mm$^2$). The specific surface area of the membrane in the dry state was 62 m$^2$/g.

## Examples 12 to 18

The mould described in example 11 was charged with mixtures the composition of which is given in Table 1; the charge was polymerized and worked up in the same way as in example 11.

Table 1

| Composition of the polymerization mixture used in the preparation of macroporous polymeric membranes and their specific surface area | | | | | | |
|---|---|---|---|---|---|---|
| Example No. | GMA (ml) | EDMA (ml) | AIBN (g) | CyOH (ml) | DoOH (ml) | S$_g$ (m$^2$/g) |
| 12 | 4,8 | 7,2 | 0,12 | 18 | 0 | 80 |
| 13 | 7,2 | 4,8 | 0,12 | 16,2 | 1,8 | 45 |
| 14 | 9,6 | 2,4 | 0,12 | 17,1 | 0,9 | 160 |
| 15 | 0,6 | 11,4 | 0,12 | 18 | 0 | 260 |
| 16 | 7,2 | 4,8 | 0,12 | 18 | 0 | 53 |
| 17 | 0,6 | 11,4 | 0,06 | 18 | 0 | 250 |
| 18 | 7,2 | 4,8 | 0,24 | 14,4 | 3,6 | 35 |
| GMA = glycidyl methacrylate; EDMA = ethylene dimethacrylate; AIBN = azo-bis(isobutyronitrile); CyOH = cyclohexanol; DoOH = dodecanol; and S$_g$ = specific surface area in the dry state, determined by the dynamic method of thermal desorption of nitrogen. | | | | | | |

## Example 19

Circular cut-outs from the macroporous membrane prepared according to example 13 were immersed into a 0,01 mol/l solution of NaOH in butanol for 24 hours. After the reaction was completed, the membranes were washed with ethanol and water and stored for further application in the wet state.

## Examples 20 and 21

The epoxy groups of the membrane of example 19 were modified with octanol or octadecanol in the same way as in example 19. In the latter case, the modification was carried out at temperature of 65 °C.

## Example 22

Circular membrane sheet with a diameter of 10 mm stamped from the membrane of example 16 were immersed for 6 hours into a 25 % commercial aqueous solution of ammonia and heated under reflux to 80 °C. The membranes were then washed with water until no ammonia could be detected in the washing water. The elemental analysis proved the presence of 1,8 mmol/g of amino groups in the modified polymer.

## Examples 23 to 30

Sheets from the membrane prepared according to example 11 were modified by the same procedure as in example 22 consisting in the reaction with an amine in pure state or in an aqueous solution carried out in a flask or a sealed ampoule. The reaction conditions and the contents of groups bound to the polymeric membranes are listed in Table 2.

Table 2

| Reaction conditions for the modification of macroporous membranes and contents of groups bound to the product | | | | | |
|---|---|---|---|---|---|
| Example Nr. | Amine | Concentration of aq.solution (% by mass) | Reaction time (h) | Temp. ($^\circ$C) | Content of groups (mmol/g) |
| 23 | dimethylamine | 100 | 3 | 60 | 2,0 |
| 24 | 2-hydroxyethylamine | 100 | 6 | 70 | 2,2 |
| 25 | bis-2-hydroxyethylamine | 100 | 6 | 70 | 2,1 |
| 26 | octylamine | 100 | 12 | 70 | 0,4 |
| 27 | ethylamine | 50 | 6 | 80 | 1,6 |
| 28 | ethylenediamine | 50 | 10 | 80 | 1,7 |
| 29 | trimethylamine•HCl | 50 | 24 | 80 | 2,2 |
| 30 | tributylamine | 100 | 24 | 80 | 0,1 |

**Example 31**

Circular sheets cut out from the macroporous membrane prepared according to example 12 were immersed for 6 hours into 0,01 mol/l sulfuric acid and heated to 80 $^\circ$C. The samples were then washed with water until no acid could be determined in the washing water, dipped into a solution of 10,2 g of NaOH in 36 ml of water, and the mixture was cooled down to 0 $^\circ$C. Under stirring of the liquid above the membranes, 24 g of propansultone were added dropwise.

After 30 minutes, the mixture was again heated to 35 $^\circ$C and the reaction continued for further 3 hours. On completion of the reaction, the mixture was allowed to stand at ambient temperature for 12 hours. The samples were washed with water, 0,5 mol/l HCl, and again with water until no acid could be detected in the washing water. In this way sulfo groups were obtained on the surface of polymeric membrane in an amount of 0,85 mmol/g.

**Example 32**

A rectangular sheet of 10 x 5 cm obtained from the plate prepared in a similar way as in example 12, the epoxy groups of which were hydrolyzed to vicinal dihydroxy groups with diluted sulfuric acid in the same way as in example 31, was washed with water and dried. The dry plate was immersed for 5 hours into a mixture of allyl glycidyl ether and dioxan (1:1 vol/vol) containing 0,3 % by volume of boron fluoride etherate and heated to 50 $^\circ$C. The grafted allyl groups were then modified with a 30 % potassium thiosulfate solution in water at ambient temperature for 24 hours, while oxygen from a cylinder was passed through the mixture every other 10 minutes for 10 minutes. After washing with water, 0,4 mol/l HCl, and again with water, the membrane contained sulfonate groups in an amount of 0,37 mmol/g as determined by acid-base titration.

**Example 33**

A circular disk with a diameter of 20 cm cut out from a square plate with a side length of 25 cm and a thickness of 3 mm was immersed into a glass dish containing 100 ml of 1,2-dichloroethane for 20 hours. Then, 45 ml of sulfur trioxide monohydrate was slowly added at room temperature under agitating by tilting the dish. The membrane was removed after one hour of reaction and washed with ethanol and water. Acid-base titration of a sample revealed that the membrane contained hydrogen sulfate groups in an amount of 1,59 mmol/g.

**Example 34**

Circular disks with a diameter of 30 mm, which were stamped from the plate prepared according to example 14, were immersed into a 10 % aqueous solution of sodium sulfide. The mixture was moderately shaken at 25 $^\circ$C for 12 hours. The disks were then washed with water until the odour of hydrogen sulfide disappeared. The modified polymer contained thiol groups in an amount of 0,52 mmol/g.

## Example 35

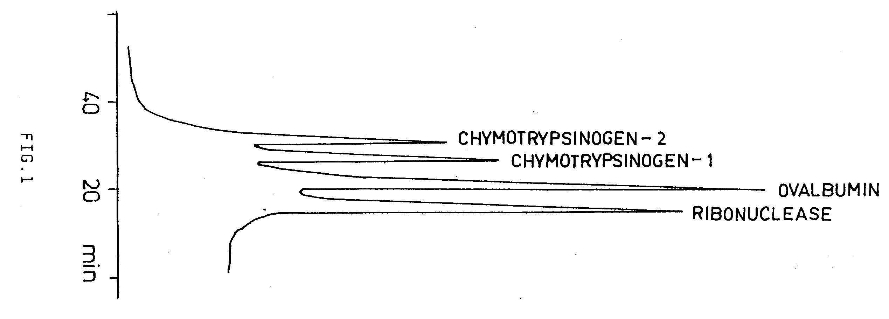

A disk with a diameter of approx. 1 cm cut from a 1-mm thick membrane prepared according to example 11 and modified according to example 19 was placed on the bottom of a vessel with a circular cross-section and a volume of about 1 ml in such a way that, after filling the vessel with liquid, the whole flow occured exclusively through the membrane. The content of the vessel was agitated with a propeller stirrer. Eluent was introduced into the vessel by a pump according to a preselected gradient. Under the membrane, resting on a support surface provided with a system of collection channels which secured a perfect and uniform outlet of eluent from every site of the membrane, also a central outlet capillary opening was provided, from which the passing eluent (or a part thereof) was led into a detector and then could be collected in order to obtain the separated compounds. The vessel was charged with a solution containing 0,1 mg of ribonuclease, 0,1 mg of ovalbumin, and 0,1 mg of chymotrypsinogen in 0,02 mol/l phosphate buffer of pH 6,8 in which ammonium sulfate was dissolved to a concentration of 2 mol/l. The same buffer containing decreasing amounts of ammonium chloride was introduced into the vessel under stirring and a pressure of 0,1 MPa at a flow rate of 0,5 ml/min. The state when the eluent contained 1 % of the original amount of ammonium sulfate was reached after 35 minutes. The eluent flowing from the vessel was led into a Gilson UV detector. The record of the detector response (chromatogram) is shown in figure 1. All originally absorbed proteins were eluted from the membrane in a yield of 100 %.

## Example 36

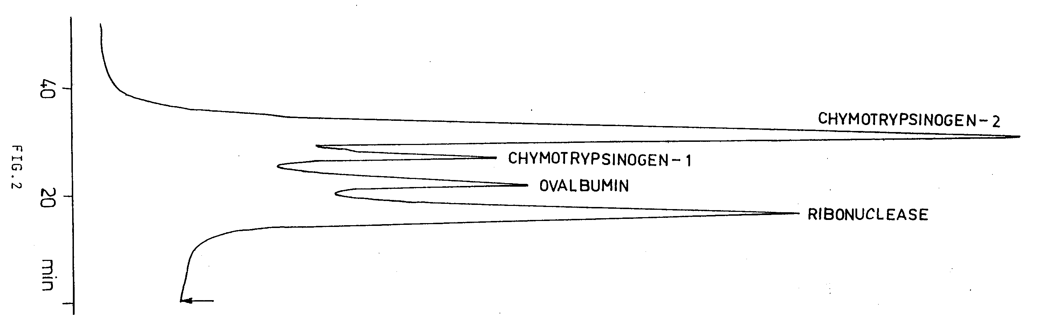

The same mixture of proteins as in example 35 was separated by the same procedure and in the same equipment, but with use of a membrane modified according to example 26. The chromatogram is shown in figure 2.

## Example 37

The same proteins as in example 35 were separated by the same procedure and in the same equipment, with the exception that the amount of each protein in the solution charged into the vessel was 5 mg, i.e. 50 time higher than in example 35. The chromatogram of the separation was absolutely the same as shown in figure 1.

## Example 38

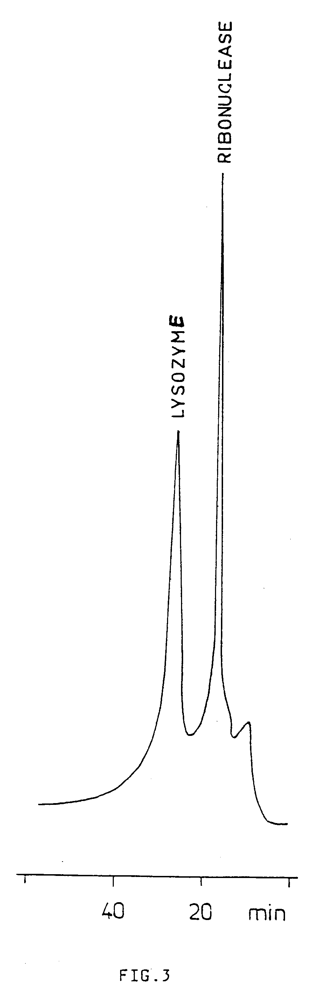

A mixture consisting of 0,1 mg of ribonuclease and 0,1 mg of lysozyme dissolved in 0,02 mol/l phosphate buffer of pH 6,8 was separated under the same conditions and in the same equipment as in example 35, but with use of a membrane made according to example 31. The elution was carried out at a flow rate of 1 ml/min and a pressure of 0,1 MPa using a gradient of the ionic strength in the same buffer, whereby the content of sodium chloride was increased so that it reached 0,5 mol/l after 15 minutes. The resulting chromatogram is shown in figure 3.

## Example 39

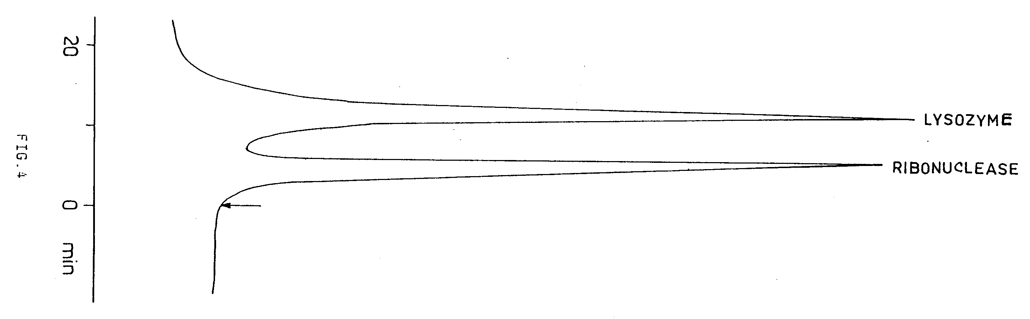

The same mixture of ribonuclease and lysozyme as in example 38 was separated under identical conditions on a membrane prepared according to example 36. The resulting chromatogram is shown in figure 4.

## Example 40

A mixture containing 150 mg of ribonuclease, 100 mg of ovalbumin, and 150 mg of chymotrypsinogen was separated in an equipment having the shape of a prism and comprising a chamber with a volume of 10 ml with a wall formed by a rectangular membrane with an area of 6 x 8 cm and a thickness of 1,5 mm prepared according to examples 11 and 19. The flow rate of the eluent was 5 ml/min at a pressure of 0,8 MPa. The elution was carried out using the same gradient of ionic strength as in example 38, and the eluent was collected in test tubes in 10 ml portions. The fifth, sixth and seventh test tubes contained 5, 85 and 10 % of the ribonuclease, respectively; the eighth, ninth and tenth test tubes contained 15, 75 and 10 % of the ovalbumin, respectively, and, the twelfth, thirteenth, fourteenth, fifteenth and sixteenth test tubes contained 3, 28, 16, 32 and 20 % of the chymotrypsinogen, respectively.

**Example 41**

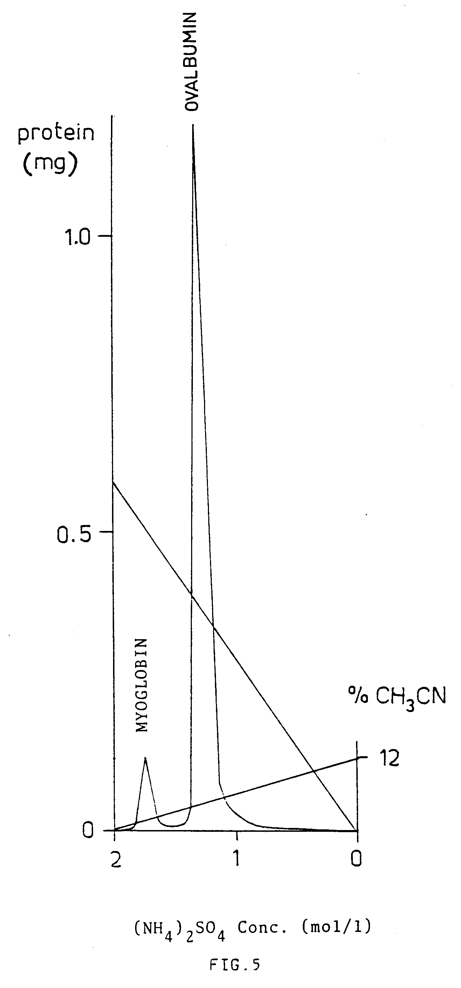

A mixture of 0,15 mg of myoglobin and 0,3 mg of ovalbumin was separated on the membrane and in the equipment according to example 35, with the exception that acetonitrile was added into the buffer with decreasing ionic strength in such amount, that its concentration was 12 % by volume after 20 minutes at a pressure of 0,25 MPa. The chromatogram of the separation is shown in figure 5 together with the ammonium sulfate and acetonitrile gradients applied.

**Claims**

1. Macroporous polymeric membranes for the separation of macromolecular substances,
   - consisting of a cross-linked polymer or copolymer on the basis of one or more monovinylic monomers selected from
        acrylates, particularly glycidyl acrylate,
        methacrylates, particularly glycidyl methacrylate,
        itaconates, particularly glycidyl itaconate,
        vinylpyridine,
        N-vinyl pyrrolidone,
        vinyl acetate,
        glycidyl vinyl ether,
        glycidyl vinyl adipate, and
        hydroxystyrene,
     and one or more cross-linking agents selected from alkylene diacrylates, particularly ethylene diacrylate, alkylene dimethacrylates, particularly ethylene dimethacrylate, hydroxyalkylene diacrylates, hydroxyalkylene dimethacrylates, divinyl pyridine and divinyl benzene,
   - and having a globular
     microstructure wherein the globular structures have a size of 0,05 to 0,5 $\mu$m and are mutually interconnected by covalent bonds, and between the globular structures being mutually communicating voids.

2. Macroporous membranes according to claim 1, characterized in that their total cross-section thickness is 0,2 to 15 mm, and the specific surface area measurable also in the dry state is up to 400 $m^2/g$.

3. Macroporous membranes according to claim 1 or 2, characterized in that they consist of a copolymer having a mass ratio of monovinylic monomer(s) to cross-linking agent(s) of from 5:95 to 95:5.

4. Macroporous membranes according to one of claims 1 to 3, characterized in that they consist of a copolymer comprising glycidyl methacrylate as monovinylic monomer in an amount corresponding to a concentration of 5 to 80 % by volume, based on the total volume of monomers and cross-linking agents present in the polymerization batch.

5. Macroporous membranes according to one of claims 1 to 4, characterized in that they consist of a copolymer comprising ethylene dimethacrylate as cross-linking agent in an amount corresponding to a concentration of 20 to 95 % by volume, based on the total volume of monomers and cross-linking agents present in the polymerization batch.

6. Macroporous membranes according to one of claims 1 to 5, characterized in that the inner surfaces comprise covalently bound allyl, hydroxy, amine, sulfonate, hydrogen sulfonate, thiol and/or $C_{1-18}$-alkyl groups.

7. Macroporous membranes according to one of claims 1 to 6, characterized in that they contain a reinforcing material or insert.

8. Method for the preparation of macroporous polymeric membranes according to one of claims 1 to 7, comprising the following steps:

- Placing a monomer mixture of one or more monovinylic
monomers selected from
acrylates, particularly glycidyl acrylate,
methacrylates, particularly glycidyl methacrylate,
itaconates, particularly glycidyl itaconate,
vinylpyridine,
N-vinyl pyrrolidone,
vinyl acetate,
glycidyl vinyl ether,
glycidyl vinyl adipate, and
hydroxystyrene,
and one or more cross-linking agents selected from
alkylene diacrylates, particularly ethylene diacrylate,
alkylene dimethacrylates, particularly ethylene dimethacrylate,
hydroxyalkylene diacrylates,
hydroxyalkylene dimethacrylates,
divinyl pyridine and
divinyl benzene,
and a polymerization initiator, preferably a radical polymerization initiator, dissolved in a porogenic inert organic solvent in a mould comprising two temperature-controlled, parallel face plates facing each other and a distance insert having a thickness corresponding to the required thickness of the membrane and being adapted to be in a sealing contact with the face plates, size and shape of the inner space of the mould defined by the face plates and the distance insert being adapted to size and shape of the desired membrane,
and
- polymerizing or copolymerizing, respectively, with heating the face plates to the polymerization or copolymerization temperature for the necessary reaction duration.

9. The method according to claim 8, characterized in that the porogenic organic solvent is selected from aliphatic and aromatic alcohols, esters of carboxylic esters, ethers, ketones, hydrocarbons, silicon oils, low-molecular weight polymers, and their mixtures.

10. The method according to claim 8 or 9, characterized in that the porogenic solvent is used in the copolymerization batch in an amount of 40 to 60 % by volume.

11. The method according to one of claims 8 to 10, characterized in that cyclohexanol or a cyclohexanol-dodecanol mixture comprising up to 20 % by volume of dodecanol is used as porogenic solvent.

12. The method according to one of claims 8 to 11, characterized in that the monovinylic monomer(s) and the cross-linking agent(s) are used in the polymerization batch in a mass ratio of from 5:95 to 95:5.

13. The method according to one of claims 8 to 12, characterized in that glycidyl methacrylate is used as monovinylic monomer in an amount of from 5 to 80 % by volume, based on the total volume of monomers and cross-linking agents present in the polymerization batch.

14. The method according to one of claims 8 to 13, characterized in that ethylene dimethacrylate is used as cross-linking agent in an amount of from 20 to 95 % by volume, based on the total volume of monomers and cross-linking agents present in the polymerization batch.

15. The method according to one of claims 8 to 14, characterized in that the obtained polymeric membranes are chemically modified by introducing ionogenic, hydrophilic and/or lyophilic groups, catalysts and/or affinants and/or other active groups or molecules.

16. The method according to claim 15, characterized in that allyl, hydroxy, amine, sulfonate, hydrogen sulfonate, thiol and/or $C_{1-18}$-alkyl groups are covalently bound to the inner surface of the membranes.

17. The method according to one of claims 8 to 16, characterized in that a reinforcing material or insert is introduced into the polymerization batch before polymerization.

**18.** Use of the macroporous polymeric membranes according to one of claims 1 to 7 for the separation and fractionation of macromolecular substances, and particularly of synthetical polymers and biopolymers.

**19.** Method for the separation of macromolecular substances, comprising the steps of:
- passing a solution of the macromolecular substances to be separated under pressure through a macroporous polymeric membrane according to one of claims 1 to 7, with sorption of the macromolecular substances in the membrane,
- eluting with an elution solvent the properties of which are changed gradually or stepwise in such a manner that individual components of the sorbed macromolecular substances are eluted, and
- detecting and/or collecting individual eluted components.

**20.** The method according to claim 19, characterized in that in the elution step the pH, the ionic strength, the temperature and/or the composition of the elution solvent are changed, particularly according to a pre-determined program.

**21.** The method according to claim 19 or 20, characterized in that a macroporous polymeric membrane according to one of claims 1 to 7 is placed and secured on a support member forming a part of the walls of a vessel comprising a liquid chamber provided with means for applying a pressure and optionally also means for stirring above the membrane, and a liquid collection system below the membrane connected to a liquid detection and/or collection system, the solution of the macromolecular substances is charged into the liquid chamber, pressure is applied to the liquid chamber, and the macromolecular components or fractions are eluted and/or collected.

**Patentansprüche**

**1.** Makroporöse polymere Membranen für die Separation von makromolekularen Stoffen,
- bestehend aus vernetzten Polymeren oder Kopolymeren auf der Basis von mono- oder mehr-vinylischen Monomeren ausgewählt aus
    Akrylaten, vorzugsweise Glyzidylakrylat,
    Methakrylaten, vorzugsweise Glyzidylmethakrylat,
    Itakonaten, vorzugsweise Glyzidylitakonat,
    Vinylpyridin,
    N-Vinylpyrrolidon,
    Vinylazetat,
    Glyzidylvinylether,
    Glyzidylvinyladipat und
    Hydroxystyrol,
    und einem oder mehreren Vernetzungsmitteln ausgewählt aus der Gruppe bestehend aus Alkylendiakrylaten, vorzugsweise Äthylendiakrylat, Alkylendimethakrylaten, vorzugsweise Äthylendimethakrylat, Hydroxyalkylendiakrylaten, Hydroxyalkylendimethakrylaten, Divinylpyridin und Divinylbenzol,
- und enthaltend eine globulare Mikrostruktur, in welcher die globularen Gebilde eine Grösse von 0,05 bis 0,5 μm haben und miteinander gegenseitig durch kovalente Bindungen gebunden sind und zwischen den globularen Gebilden kommunizierende freie Räume sind.

**2.** Makroporöse polymere Membranen nach Anspruch 1, dadurch gekennzeichnet, dass ihre gesamte Dicke im Querschnitt 0,2 bis 15 mm und die spezifische Oberfläche, messbar auch in trockenem Zustand, bis zu 400 $m^2$/g beträgt.

**3.** Makroporöse polymere Membranen nach den Ansprüchen 1 oder 2, dadurch gekennzeichnet, dass sie aus einem Polymeren bestehen welches ein Massenverhältnis des(r) monovinylischen Monomeren zu dem(n) Vernetzungsmitteln im Bereich von 5 : 95 bis 95 : 5 Gew. % hat.

**4.** Makroporöse polymere Membranen nach einem der Ansprüche 1 bis 3; dadurch gekennzeichnet, dass sie aus einem Kopolymeren bestehen, dass als monovinylisches Monomer das Glyzidylmethakrylat in einer Menge, entsprechend der Konzentration von 5 bis 80 Vol. %, bezogen auf das gesamte Volumen, der in dem Polymerisationsansatz anwesenden Monomeren und der Vernetzungsmittel, enthaltet.

EP 0 320 023 B1

5. Makroporöse polymere Membranen nach einem der Ansprüche 1 bis 4, dadurch gekennzeichnet, dass sie aus einem Kopolymeren bestehen, welches als Vernetzungsmittel das Äthylendimethakrylat in einer Menge, entsprechend der Konzentration von 20 bis 95 Vol %, bezogen auf das gesammte Volumen der, in dem Polymerisationsansatz anwesenden Monomeren und Vernetzungsmittel, enthaltet.

6. Makroporöse polymere Membranen nach einem der Ansprüche 1 bis 5, dadurch gekennzeichnet, dass sie auf der inneren Oberfläche kovalent gebundene Allyl-, Hydroxyl-, Amin-, Sulfonat-, Hydrogensulfonat-, Sulfhydrid-, und/oder $C_{1-18}$ Alkylgruppen, enthalten.

7. Makroporöse polymere Membranen nach einem der Ansprüche 1 bis 6, dadurch gekennzeichnet, dass sie ein armierendes Material oder Einlage enthalten.

8. Verfahren zur Herstellung der makroporösen polymeren Membranen nach einem der Ansprüche 1 bis 7, durch

 - Einbringen eines Monomerengemisches der mono- oder mehr- vinylischen Monomeren, ausgewählt aus

    Akrylaten, vorzugsweise Glyzidylakrylat,
    Methacrylaten, vorzugsweise Glyzidylmethakrylat,
    Itakonaten, vorzugsweise Glyzidylitakonat,
    Vinylpyridin,
    N-Vinylpyrrolidon,
    Vinylacetat und
    Hydroxystyrol

und eines oder mehrerer Vernetzungsmittel, ausgewählt aus

    Alkylendiakrylaten, vorzugsweise Äthylendiakrylat,
    Alkylendimethakrylaten, vorzugsweise Äthylendimethakrylat,
    Hydroxyalkylendiakrylaten,
    Hydroxyalkylendimethakrylaten,
    Divinylpyridin und Divinylbenzol,

und eines Polymerisationsinitiatoren, mit Vorteil eines radikalischen Initiators, aufgelöst in einem porogenen organischen Lösungsmittel in einer Form, gebildet durch zwei temperierte paralelle Frontplatten und eine Distanzeinlage, deren Stärke der geforderten Dicke der Membrane entspricht und die so angepasst ist, dass sie im dichtschliessenden Kontakt mit den Frontplatten ist, die Grösse und Form des inneren Raumes der Form, definiert durch die Frontplatten und die Distanzeinlage, angepasst der Grösse und der Form der gewünschten Membrane, und

 - die Polymerisation oder Kopolymerisation, respektive dass Erwärmen der Frontplatten auf die Polymerisations- oder Kopolymerisationstemperatur für die notwendige Reaktionsdauer.

9. Verfahren nach Anspruch 8, dadurch gekennzeichnet, dass das porogene organische Lösungsmittel ausgewählt ist aus alifatischen und aromatischen Alkoholen, Karboxylsäureestern, Äthern, Ketonen, Kohlenwasserstoffen, Silikonölen, niedrigmolekularen Polymeren und ihren Gemischen.

10. Verfahren nach Anspruch 8 oder 9, dadurch gekennzeichnet, dass das porogene Lösungsmittel in dem Polymerisationsansatz 40 bis 60 Vol. % beträgt.

11. Verfahren nach einem der Ansprüche 8 bis 10, dadurch gekennzeichnet dass, das Zyklohexanol oder ein Zyklohexanol-Dodekanolgemisch, enthaltend bis zu 20 Vol. % Dodekanol, als porogenes Lösungsmittel, verwendet wird.

12. Verfahren nach einem der Ansprüche 8 bis 11, dadurch gekennzeichnet, dass das monovinylische Momomer(en) und das (die) Vernetzungsmittel in dem Polymerisationsansatz in eimen Gewichtsverhältnis von 5:95 bis 95:5, angewendet wird.

13. Verfahren nach einem der Ansprüche 8 bis 12, dadurch gekennzeichnet, dass das Glyzidylmethakrylat als monovinylisches Monomer, in einer Menge von 5 bis 80 Vol.%, bezogen auf das gesamte Volumen der Monomeren und der Vernetzungsmittel, anwesend in dem Polymerisationsansatz, angewendet wird.

14

**14.** Verfahren nach einem der Ansprüche 8 bis 13, dadurch gekennzeichnet, dass das Äthylendimethakrylat als Vernetzungsmittel in einer Menge von 20 bis 95 Vol.%, bezogen auf das gesamte Volumen der Monomeren und der Vernetzungsmittel, anwesend in dem Polymerisationsansatz, angewendet wird.

**15.** Verfahren nach einem der Ansprüche 8 bis 14, dadurch gekennzeichnet, dass die gewonnenen polymeren Membranen chemisch modifiziert sind durch das Einführen von ionogenen, hydrophilen und/oder lyophilen Gruppen, Katalysatoren und/oder Affinanten und/oder anderen aktiven Gruppen oder Molekülen.

**16.** Verfahren nach Anspruch 15, dadurch gekennzeichnet, dass Allyl-, Hydroxy-, Amin-, Sulphonat-, Hydrogensulphonat-, Sulfhydrid- und/oder $C_{1-18}$ Alkylgruppen kovalent zu der inneren Oberfläche, gebunden sind.

**17.** Verfahren nach einem der Ansprüche 8 bis 16, dadurch gekennzeichnet, dass ein armierendes Material oder Einlage in den Polymerisationsansatz vor der Polymerisation eingelegt wird.

**18.** Anwendung der makroporösen polymeren Membranen nach einem der Ansprüche 1 bis 7 für die Trennung und Fraktionierung von makromolekularen Stoffen, und vorzugsweise von synthetischen Polymeren und Biopolymeren.

**19.** Verfahren zur Trennung von makromolekularen Stoffen enthaltend die folgenden Stufen:
- den Durchfluss einer Lösung des zu trennenden makromolekularen Stoffes unter Druck durch die polymere Membrane nach einem der Ansprüche 1 bis 7, mit der Sorption des makromolekularen Stoffes in der Membrane,
- die Elution mit einem Elutionsmittel, dessen Eigenschaften sich sukzessive oder stufenweise so ändern, dass die einzelnen Komponenten des sorbierten makromolekularen Stoffes eluiert werden, und
- die Detektion und/oder Sammlung der einzelnen eluierten Komponenten.

**20.** Verfahren nach Anspruch 19, dadurch gekennzeichnet, dass in der Elutionsstufe der pH-Wert, die Ionenstärke, die Temperatur und/oder die Zusammenstzung des Elutionsmittels geändert wird, vorzugsweise nach dem im vorhinein gegebenen Program.

**21.** Verfahren nach den Ansprüchen 19 oder 20, dadurch gekennzeichnet, dass eine makroporöse polymere Membrane nach einem der Ansprüche 1 bis 7, plaziert und gesichert wird auf einer Unterlage, welche einen Teil der Wände eines Gefässes bildet, dass eine Flüssigkeitskammer, versehen mit Mitteln zur Anwendung von Druck und wählbar, auch mit Mitteln für das Mischen oberhalb der Membrane, und einem System zum Sammeln der Flüssigkeit unterhalb der Membrane enthaltet, die Lösung der makromolekularen Stoffe in die Flüssigkeitskammer gefüllt wird, in der Flüssigkeitskammer Druck angewendet wird und die makromolekularen Komponenten oder Fraktionen eluiert und/oder gesammelt werden.

**Revendications**

**1.** Membranes polymériques macroporeuses pour la séparation de substances macromoléculaires,
- consistant d'un polymère ou copolymère réticulé produit sur la base d'un ou plusieurs monomères monovinyliques choisis parmi les
    acrylates, en particulier glycidyle acrylate,
    méthacrylates, en particulier glycidyle méthacrylate,
    itaconates, en particulier glycidyle itaconate,
    vinylpyridine,
    N-vinyl pyrrolidone,
    vinyle acétate,
    glycidyle vinyle éther,
    glycidyle vinyle adipate,
    et
    hydroxystyrène,

avec un ou plusieurs agents de réticulation choisis parmi les alkylène diacrylates, en particulier éthylène diacrylate, alkylène diméthacrylates, en particulier éthylène diméthacrylate, hydroxyalkylène diacrylates, hydroxyalkylène diméthacrylates, divinyle pyridine et divinyle benzène,

- et ayant une microstructure globulaire dans laquelle les structures globulaires ont des dimensions entre 0,05 et 0,5 $\mu$m et sont mutuellement liées par des liaisons covalentes et les espaces vides entre les structures globulaires étant mutuellement interconnectés.

2. Membranes macroporeuses selon la revendication 1 caractérisées par leur épaisseur de section totale entre 0,2 et 15 mm, et par leur surface spécifique qui, mesurée également à l' état sec, est de valeur jusqu'à 400 m$^2$/g.

3. Membranes macroporeuses selon la revendication 1 ou 2 caractérisées par le fait qu'elles consistent d'un copolymère ayant le rapport de masses molaires du(des) monomère(s) monovinilique(s) au(x) agent(s) de réticulation entre 5:95 et 95:5.

4. Membranes macroporeuses selon une des revendications 1 à 3 caractérisées par le fait qu'elles consistent d'un copolymère contenant du glycidyle méthacrylate comme monomère monovinylique dans une quantité correspondant à une concentration de 5 à 80% volumétriques, sur la base du volume total de monomères et agents de réticulation présents dans le mélange de polymérisation.

5. Membranes macroporeuses selon une des revendications 1 à 4 caractérisées par le fait qu'elles consistent d'un copolymère contenant du éthylène diméthacrylate comme agent de réticulation dans une quantité correspondant à une concentration de 20 à 95% volumétriques, sur la base du volume total des monomères et agents de réticulation présents dans le mélange de polymérisation.

6. Membranes macroporeuses selon une des revendications 1 à 5, caractérisées par le fait que les surfaces intérieures contiennent des groupes liés par liaisons covalentes qui sont des groupes allyle, hydroxy, amine, sulfonate, hydrogène sulfonate, thiol et/ou C$_{1-18}$-alkyle.

7. Membranes macroporeuses selon une des revendications 1 à 6 caractérisées par le fait qu'elles contiennent un matériau ou une inclusion de renforcement.

8. Méthode de préparation des membranes macroporeuses polymériques selon une des revendication 1 à 7 consistant en les étapes suivantes:
   - placement d'un mélange d' un ou plusieurs monomères monovinyliques choisis parmi les
      acrylates, en particulier glycidyle acrylate,
      méthacrylates, en particulier glycidyle méthacrylate,
      itaconates, en particulier glycidyle itaconate,
      vinylpyridine,
      N-vinyl pyrrolidone,
      vinyle acétate,
      glycidyle vinyle éther,
      glycidyle vinyle adipate,
      et
      hydroxystyrène,
   avec un ou plusieurs agents de réticulation choisis parmi les
      alkylène diacrylates, en particulier éthylène diacrylate,
      alkylène diméthacrylates, en particulier éthylène diméthacrylate,
      hydroxyalkylène diacrylates,
      hydroxyalkylène diméthacrylates,
      divinyle pyridine et
      divinyle benzène,
   et avec un initiateur de polymérisation, de préférence un initiateur de polymérisation radicalaire dissous dans un solvant organique porogénique indifférant
   dans un moule construit avec deux plaques parallèles pouvant être chauffées à une température contrôlée et positionnées à une distance l'une de l'autre correspondant à l'épaisseur voulue de la membrane à l'aide d'un joint intercalaire qui assure l'étanchéité du moule et dont les dimensions et la forme correspondent aux dimensions et la forme de la membrane désirée,

EP 0 320 023 B1

et

- polymérisation ou copolymérisation en chauffant les plaques du moule à la température de polymérisation ou copolymérisation pendant la durée de réaction nécessaire.

9. La méthode selon la revendication 8 caractérisée par le fait que le solvant porogénique organique est choisi parmi les alcools aliphatiques et aromatiques, les esters d'acides carboxyliques, les éthers, kétones, hydrocarbures, huiles de silicone, polymère de petite masse moléculaire, et leurs mélanges.

10. La méthode selon une des revendications 8 au 9, caractérisée par le fait que le solvant porogénique est utilisé dans le mélange de réaction dans une quantité de 40 à 60% volumétriques.

11. La méthode selon une des revendications 8 à 10, caractérisée par le fait que l'on utilise comme solvant porogénique du cyclohexanole ou le mélange du cyclohexanole et du dodécanole avec une proportion du dodécanole jusqu'à 20%.

12. La méthode selon une des revendications 8 à 11 caractérisée par le fait que le(s) monomère(s) monovinylique(s) et l'(es) agent(s) de réticulation son utilisées dans le mélange do polymérisation dans un rapport de masses entre 5:95 et 95:5.

13. La méthode selon une des revendications 8 à 12 caractérisée par le fait que le glycidyle méthacrylate est utilisé comme monomère monovinylique dans une quantité entre 5 et 80% volumétriques, sur la base du volume total des monomères et agents de réticulation présents dans le mélange de polymérisation.

14. La méthode selon une des revendications 8 à 13 caractérisée par le fait que l'éthylène diméthacrylate est utilisé comme agent de réticulation dans une quantité entre 20 et 95% volumétriques, sur la base du volume total de monomères et agents de réticulation dans le mélange de réaction.

15. La méthode selon une des revendications 8 à 14 caractérisée par le fait que le membranes polymériques obtenues sont modifiées chimiquement en introduisant des groupes ionogéniques, hydrophiliques et/ou lyophiliques, des agents catalytiques et/ou de raffinement et/ou autres groupes actifs ou molécules.

16. La méthode selon la revendication 15 caractérisée par le fait que des groupes allyl, hydroxy, amine, sulfonate, hydrogène sulfonate, thiol et/ou $C_{1-18}$-alkyle sont liés par liaison covalente à la surface intérieure des membranes.

17. La méthode selon une des revendications 8 à 16 caractérisée par le fait qu'un matériau ou inclusion de renforcement sont introduits dans le mélange de polymérisation avant la polymérisation.

18. L'utilisation de membranes macroporeuses selon une des revendications 1 à 7 pour la séparation et fractionation des substances macromoléculaires, en particulier de polymères synthétiques et biopolymères.

19. Méthode de séparation de substances macromoléculaires consistant des étapes suivantes:
- passage sous pression d'une substance macromoléculaire qui doit être séparée par une membrane macroporeuse polymérique selon une des revendications 1 à 7 avec sorption de la substance macromoléculaire dans la membrane,
- élution avec un solvant d'élution dont les propriétés sont changées graduellement ou par saut de façon à ce que les composantes individuelles de la substance macromoléculaire absorbée soient relâchées,
et
- détection et/ou collecte des composantes individuelles de l'élution.

20. La méthode selon la revendication 19 caractérisée par le fait que pendant l'étape de l'élution, le pH, la force ionique, le température et/ou la composition du solvant d'élution sont changés, en particulier conformément à un programme prédéterminé.

17

**21.** La méthode selon une des revendications 19 ou 20 caractérisée par le fait qu'une membrane macroporeuse polymérique selon une des revendications 1 à 7 est placée et fixée sur un support qui fait partie des parois du récipient lequel comprend un réservoir du liquide équipé pour appliquer la pression et, en option, d'un moyen d'agitation au dessus de la membrane, et un système de collecte du liquide en dessous de la membrane lié à un système de détection et/ou collecte du liquide, la solution de substances macromoléculaire est introduite dans le réservoir au liquide, la pression est appliquée et les composantes macromoléculaires ou fractions sont éluées et/ou collectées.

FIG.1

FIG.2

FIG.3

EP 0 320 023 B1

FIG.4

FIG.5